# EUROPÄISCHE PATENTSCHRIFT

(11) **EP 0 325 969 B1**
(45) Veröffentlichungstag und Bekanntmachung des Hinweises auf die Patenterteilung: **14.04.1993**
(21) Anmeldenummer: 89100600.9
(22) Anmeldetag: 13.01.1989
(51) Int. Cl.: A61K 7/06

(54) **Verwendung eines Gemisches zur Behandlung der Alopezie**
Use of a mixture for treatment of alopecia
Utilisation d'un mélange pour le traitement de l'alopécie

(30) Priorität: 15.01.1988 DE 3801034
(43) Veröffentlichungstag der Anmeldung: 02.08.1989
(73) Patentinhaber: DR. AUGUST WOLFF CHEMISCH-PHARMAZEUTISCHE FABRIK GMBH & CO. KG, D-33611 Bielefeld (DE)
(72) Erfinder: Brenner, Günther, Dr., D-4802 Halle (DE)
(74) Vertreter: Grünecker, Kinkeldey, Stockmair & Schwanhäusser Anwaltssozietät

(56) Entgegenhaltungen:
- DE-C- 835 038

## Beschreibung

Die Erfindung betrifft die Verwendung eines Gemisches auf der Basis einer synergistisch bzw. in Teilbereichen auch antagonistisch wirkenden Kombination zur Herstellung eines Arzneimittels zur Behandlung der androgenetischen Alopezie.

Eine Lokaltherapie des androgenetisch bedingten Haarausfalles des Mannes und der Frau im Sinne der Erhaltung eines Terminalhaares ist theoretisch durch mehrere Wirkprinzipien möglich und wird in der Praxis mit mehr oder weniger großem, meistens jedoch unbefriedigendem Erfolg durchgeführt.

Der androgenetischen Alopezie des Mannes liegt eine erblich determinierte Anlage, auf männliche Sexualhormone mit einem beschleunigten Haarzyklus zu reagieren, zugrunde. Die androgenetische Alopezie der Frau beruht ebenfalls auf einer verstärkten androgenen Stimulation der Haarfollikel, die besonders in der postklimakterischen Phase durch den Abfall des Estrogenspiegels verstärkt auftritt.

Eine Möglichkeit zur Haarerhaltung bei der androgenetischen Alopezie durch Einflußnahme auf den zeitlichen Ablauf des physiologischen Haarzyklus besteht nach STÜTTGEN und SCHÄFER (Ärztl. Kosmetologie 7, 131-133 (1977)) durch die gezielte Beeinflussung des intrazellulären "second messenger" cAMP-Systems, welches aus ATP mittels des Enzyms Adenylcyclase entsteht und unter Einfluß einer Phosphodiesterase wieder inaktiviert wird. Durch diese beiden Enzyme erfolgt die Regulation des interzellulären cAMP-Spiegels:

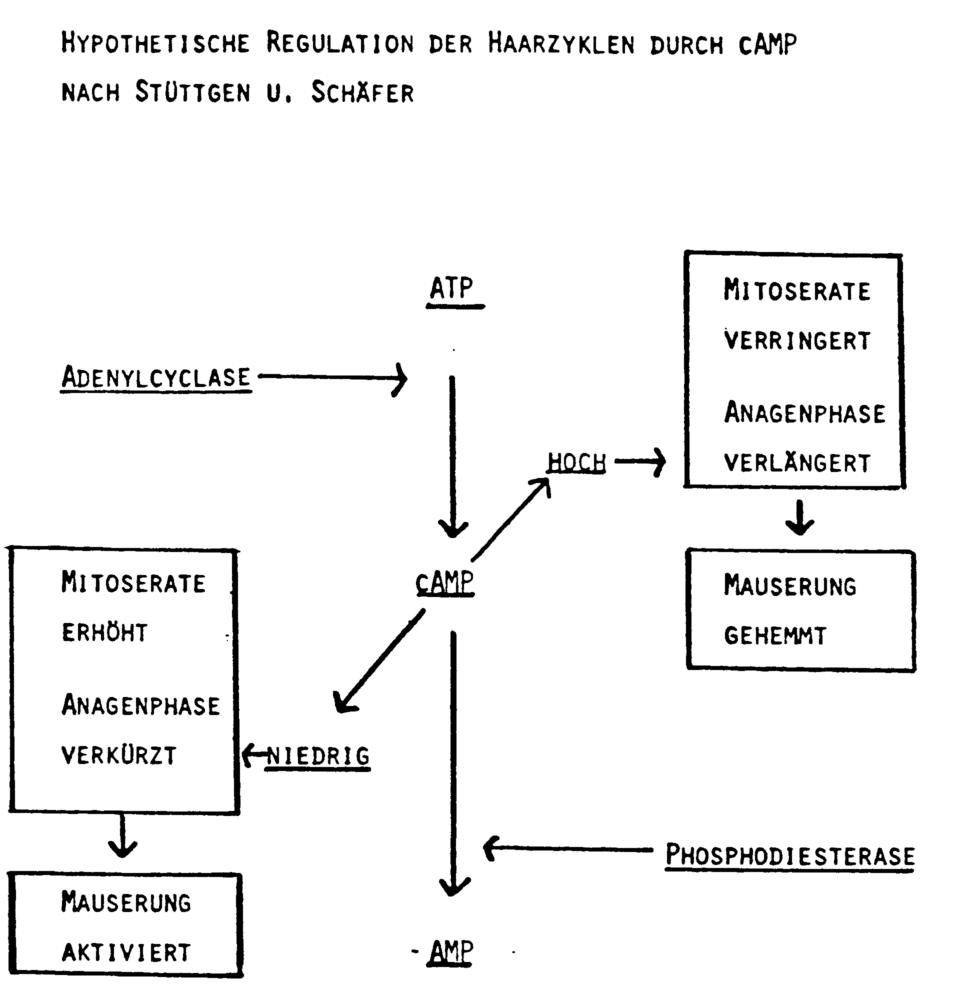

Unter dem Einfluß von Androgenen bzw. des aktiven Metaboliten 5-α-Dihydrotestosterons, welcher aus Testosteron durch das Enzym 5-α-Reduktase gebildet wird, kommt es zu einer Hemmung der Aktivität der Adenylcyclase und somit zu einer Verhinderung bzw. Reduzierung der cAMP-Bildung im Haarfollikel. Dieses wiederum führt zu einer Verkürzung der Anagenphase und als Konsequenz zu einer erhöhten Mitoserate innerhalb des Haarzyklus.

Da jedes Haar nur eine bestimmte Anzahl von Wachstumszyklen durchlaufen kann, bevor es durch Erschöpfung des Follikels ausfällt, bedeutet eine durch gezielte Beeinflussung des cAMP-Systems bewirkte Verlängerung der Anagenphase und damit des gesamten Haarzyklus (reduzierte Mitoserate) eine Möglichkeit der Behandlung der androgenetischen Alopezie im Sinne der Haarerhaltung.

Eine derartige Beeinflussung des cAMP-Systems kann auf verschiedene Weise erfolgen:
1. Aufhebung der durch 5-α-Dihydrotestosteron bewirkten Hemmung der Adenylcyclase durch Antiandrogene, wie Cyproteronacetat, 17-α-Hydroxyprogesteron, Spironolacton oder 17-β-Estradiol, und zwar entweder durch direkte Hemmung der 5-α-Reduktase oder durch Blockierung von 5-α-Dihydrotestosteron-spezifischen Rezeptoren.
2. Ein Anstieg des intrazellulären cAMP-Spiegels kann aber auch erreicht werden über eine Hemmung der Phosphodiesterase durch Methylxanthine, wie Coffein und Theophyllin. Eine derartige Einflußnahme auf das cAMP-System durch nichthormonelle, in ihrer Toxizität einschätzbare Wirkstoffe ist als Alternative zu der unter Punkt 1 beschriebenen Hormontherapie, die besonders beim Mann mit zum Teil nicht unbedenklichen Nebenwirkungen behaftet ist, entschieden zu bevorzugen.

Die Wirkung von Coffein als dem stärksten Hemmstoff der Phosphodiesterase - auch an der menschlichen Kopfhaut - ist aus Ärzt. Kosmetologie 14, 209-220 (1984) bekannt.

Ärztliche Kosmetologie 10, 163-187 (1980) beschreibt die Verwendung eines Gemisches aus Nicotinsäurebenzylester und Coffein in Salbengrundlage zur Behandlung von Hautkrankheiten.

Die vorliegende Erfindung betrifft die Verwendung eines Gemisches, enthaltend 0,01 bis 1,0 Gew.-% mindestens eines Methylxanthins und/oder mindestens eines seiner Derivate und 0,1 bis 0,25 Gew.-% mindestens eines Nicotinsäureesters sowie übliche Träger und/oder Verdünnungsmittel, wobei keine hormonellen Wirkstoffe enthalten sind, zur Herstellung eines Arzneimittels zur Behandlung der androgenetischen Alopezie.

Das erfindungsgemäß verwendete Gemisch besitzt eine synergistische und auch antagonistische Wirkung. Die Mitoserate des Kopfhaares wird durch eine biochemische Beeinflussung des zeitlichen Ablaufes der Haarzyklen verringert. Desweiteren wird die Durchblutung der Kopfhaut gefördert und die Seborrhoe sowie die Schuppenbildung reduziert.

Als Methylxanthin wird vorzugsweise Theophyllin oder Coffein verwendet, wobei Coffein (1,3,7-Trimethylxanthin) besonders bevorzugt ist.

Neben der beschriebenen biochemischen Wirkung des Coffeins auf das cAMP-System bewirkt Coffein auch eine Vasodilatation, d.h. eine Erweiterung der Blutgefäße besonders im Bereich der Mikrozirkulation. Durch diese durchblutungsfördernde Wirkung werden den Haarfollikeln wieder vermehrt Nährstoffe und Sauerstoff zugeführt, welches besonders bei der androgenetischen Alopezie durch eine zu starke Straffung der Kopfhaut und der damit oft verbundenen Minderdurchblutung von großer therapeutischer Relevanz ist. Desweiteren besitzt das Coffein auch eine ausgeprägte Antischuppenwirkung.

Als Nicotinsäureester ist Nicotinsäurebenzylester (Benzylnicotinat) bevorzugt. Nicotinsäurebenzylester ist eine Substanz, die ebenfalls und zwar in noch stärkerem Maße nach topischer Applikation die Durchblutung besonders im Kapillargebiet erhöht und so in Kombination mit dem Methylxanthin oder seinem Derivat synergistische Wirkung zeigt. Aufgrund seiner Lipoidlöslichkeit durchdringt Nicotinsäurebenzylester rasch die Hornschicht und wird innerhalb der lebenden Hautschichten durch Esterasen zu Nicotinsäure hydrolysiert. Seine intensive durchblutungsfördernde Wirkung im Kapillargebiet führt zu einer Zunahme der Blutmenge im behandelten Bereich auf der Kopfhaut (Erythembildung, Flush), die über mehrere Stunden anhalten kann.

Aufgrund seines großen biochemischen Wirkungsspektrums als Vorläufer der Pyridinnukleotide (Koenzyme der Dehydrogenase) beeinflußt Nicotinsäure indirekt eine Reihe von Stoffwechselreaktionen, die auch bei der Pathogenese der androgenetischen Alopezie eine Rolle spielen könnten.

Da bei der androgenetischen Alopezie sehr oft eine sogenannte "Begleitseborrhoe", d.h. eine Vermehrung der Kopfhaut- und Haarlipide vorliegt, kommt eine weitere Eigenschaft der Nicotinsäure - nämlich ihre lipolysehemmende Wirkung - in dem beschriebenen Haar- und Kopfhauttherapeutikum zum Tragen: Da die Lipolyse (Spaltung der Triglyceride in Fettsäuren und Glycerin) in den Talgdrüsen und der behaarten Kopfhaut durch Mikroben verursacht wird, bieten sich grundsätzlich zwei therapeutische Wege an, die hohen Fettsäureanteile zu reduzieren:
1) Eine Hemmung der Lipolyse durch antimikrobielle Substanzen, wie Isopropanol, das auch bei dem erfindungsgemäß verwendeten Gemisch als Verdünnungsmittel verwendet werden kann.
2) Eine biochemische Beeinflussung der Lipolyse durch die gezielte Hemmung der Triglyceridlipase durch Nicotinsäure: Die Nicotinsäure ist einer der potentesten Hemmstoffe für die Triglyceridlipase.

Es wurde nun gefunden, daß Nicotinsäure diese Hemmwirkung auf die Triglyceridlipase auch in Gegenwart von Methylxanthinen oder Derivaten davon und einem erhöhten cAMP-Spiegel vorrangig ausübt, da ihr Angriffspunkt anscheinend "lipasenäher" liegt. Somit antagonisiert die Nicotinsäure in Kombination mit dem Methylxanthin oder einem seiner Derivate nicht die im Sinne des Therapiezieles erwünschte Erhöhung des cAMP-Spiegels im Haarfollikel - wohl aber die sekundäre Aktivierung der Triglyceridlipase in der Talgdrüse und die dadurch induzierte Freisetzung von Fettsäuren.

Das Methylxanthin und/oder eines seiner Derivate wird in einer Menge von 0,1 bis 1,0 Gew.-% und der Nicotinsäureester in einer Menge von 0,1 bis 0,25 Gew.-%, jeweils bezogen auf das Gemisch, verwendet.

Neben den vorstehenden Verbindungen kann das erfindungsgemäß verwendete Gemisch weitere übliche Zusätze, wie Puffersubstanzen, Stabilisatoren oder Parfumöle, enthalten.

Ein Symptom, welches oft bei der androgenetischen Alopezie neben dem Haarausfall und der Begleitseborrhoe auftritt, ist eine vermehrte Kopfschuppenbildung. Kopfschuppen stellen das sichtbare Equivalent einer erhöhten Mitoserate in der Epidermis dar. Daraus resultiert ein erhöhter "Turnover" von epidermalen Zellen, die nach Durchwanderung der lebenden Epidermis und der Hornschicht als große Zellkomplexe abgestoßen werden. Die Behandlung vermehrter Kopfschuppenbildung kann durch eine Reduzierung der erhöhten Mitoserate und durch Anwendung von Stoffen mit keratolytischer Wirkung erfolgen.

Salicylsäure ist ein bewährtes Keratolytikum. Vorzugsweise enthält das erfindungsgemäß verwendete Gemisch deshalb weiterhin Salicylsäure, insbesondere in einer Menge von 0,5 Gew.-%, bezogen auf das Gemisch.

Das erfindungsgemäß verwendete Gemisch enthält übliche Träger- und/oder Verdünnungsmittel. Als Verdünnungsmittel werden vorzugsweise Ethanol und/oder Isopropanol verwendet. So kann das erfindungsgemäß verwendete Gemisch beispielsweise 30 bis 70 % Ethanol und/oder Isopropanol und als Rest Wasser enthalten.

Das erfindungsgemäß verwendete Haar- und Kopfhautbehandlungsmittel wurde an männlichen und weiblichen Patienten bei der Prüfindikation androgenetische Alopezie klinisch untersucht:
Prüfkriterien waren einmal subjektive Symptome, wie Schmerzen und Juckreiz im Bereich des Haarbodens und Stärke des Haarausfalles, und objektive Symptome, wie Seborrhoe, Kopfschuppen und Ausmaß der Haarlichtung, ferner die quantitative Beurteilung der Haarwachstumskapazität sowie Bestimmung der Haardichte.

### Material und Methoden:

An den Untersuchungen nahmen 8 Patienten teil, die an androgenetischer Alopezie litten. Bei 7 Männern im Alter von 22 bis 28 Jahren, sowie bei einer Frau im Alter von 67 Jahren erstreckte sich die Behandlung mit dem erfindungsgemäß verwendeten Gemisch über 6 Monate. Während dieser Zeit kamen keine zusätzlichen äußerlichen oder innerlichen Haarbehandlungsmittel zur Anwendung.

Die Haarwäsche erfolgte nach kosmetischen Bedürfnissen der einzelnen Probanden so oft wie nötig mit dem gleichen Haarwaschmittel.

Vor Behandlung, nach 3-monatiger Behandlung und nach 6-monatiger Behandlung wurden zum einen subjektive Angaben der Patienten über Schmerzen und Juckreiz im Bereich des Haarbodens sowie über die Intensität der Effluviums (Haarausfalles) geprüft. Zum anderen wurden vor, während und nach Therapie objektive Symptome, wie Seborrhoe, Kopfschuppen und Haarlichtung in ihrem Ausmaß immer durch denselben Untersucher beurteilt. Ferner wurde zu Beginn der Behandlung, nach 3-monatiger und nach 6-monatiger Therapie das Haarwurzelmuster für die frontale und occipitale Region des Capillitiums bestimmt (Trichogramme). Darüberhinaus wurde die Anzahl der Haare in einem kreisförmigen Areal von 2,5 cm Durchmesser in der Parieto-Occipitalregion bestimmt. Mit Hilfe einer Schablone wurde gewährleistet, daß die Zählung der Haare immer in demselben Areal vorgenommen wurde.

### Ergebnisse

### Subjektive Symptome.

Alle Patienten berichteten über eine Reduzierung der Schmerzen und des Juckreizes im Bereich des Haarbodens. 7 Patienten verzeichneten eine Besserung in der Intensität des Haarausfalles.

### Objektive Symptome.

Bei allen Patienten konnte eine deutliche Abnahme einer vor Behandlungsbeginn bestehenden Seborrhoe beobachtet werden, ferner war ein Nachlassen einer vor Behandlung bestehenden Kopfschuppung zu verzeichnen. Bezogen auf die Stadieneinteilung der androgenetischen Alopezie nach Hamilton konnte während des Untersuchungszeitraumes keine Veränderung nachgewiesen werden.

### Objektiv-quantitative Messungen des Haarwachstums.

### Haarwurzelmuster.

Trichogrammkontrollen, zu Beginn der Behandlung, nach 3-monatiger und nach 6-monatiger Therapie zeigten bei 3 Patienten keine wesentlichen Veränderungen. Bei 5 Patienten kam es jedoch nach 6-monatiger Behandlung zu einer teilweisen deutlichen Zunahme des prozentualen Anteils anagener Haare und zu einer Abnahme des prozentualen Anteils von Telogenhaaren.

### Haardichte.

Die Anzahl der Haare in einem kreisförmigen Areal von 2,5 cm Durchmesser in der Parieto-Occipitalregion ergab bei allen bisher abgeschlossenen 8 Behandlungsfällen eine Zunahme der Anzahl der Haare.

## Patentansprüche

1. Verwendung eines Gemisches, enthaltend 0,1 bis 1,0 Gew.-% mindestens eines Methylxanthins und/oder mindestens eines seiner Derivate und 0,1 bis 0,25 Gew.-% mindestens eines Nicotinsäureesters sowie übliche Träger und/oder Verdünnungsmittel, wobei keine hormonellen Wirkstoffe enthalten sind, zur Herstellung eines Arzneimittels zur Behandlung der androgenetischen Alopezie.

2. Verwendung nach Anspruch 1, **dadurch gekennzeichnet,** daß das Methylxanthin Theophyllin oder Coffein ist.

3. Verwendung nach Anspruch 1 oder 2, **dadurch gekennzeichnet,** daß der Nicotinsäureester Nicotinsäurebenzylester ist.

4. Verwendung nach mindestens einem der Ansprüche 1 bis 3, **dadurch gekennzeichnet,** daß das Gemisch weiterhin Puffersubstanzen, Stabilisatoren und Parfumöle enthält.

5. Verwendung nach mindestens einem der Ansprüch 1 bis 4, **dadurch gekennzeichnet,** daß das Gemisch weiterhin Salicylsäure enthält.

6. Verwendung nach Anspruch 5, **dadurch gekennzeichnet,** daß das Gemisch 0,5 Gew.-% Salicylsäure enthält.

7. Verwendung nach mindestens einem der Ansprüche 1 bis 6, **dadurch gekennzeichnet,** daß das Gemisch als Verdünnungsmittel Ethanol und/oder Isopropanol enthält.

## Claims

1. Use of a mixture containing from 0.1 to 1.0 parts by weight of at least one methylxanthine and/or at least one of its derivatives and from 0.1 to 0.25 parts by weight of at least one nicotinic acid ester as well as conventional carriers and/or diluents without containing any active hormone substances, for the production of a medicament for the treatment of androgenetic alopecia.

2. Use according to claim 1 **characterized in that** said methylxanthine is theophylline or caffeine.

3. Use according to claim 1 or 2 **characterized in that** the nicotinic acid ester is nicotinic acid benzylester.

4. Use according to at least one of claims 1 to 3 **characterized in that** the mixture further contains buffer substances, stabilisers and perfume oils.

5. Use according to at least one of claims 1 to 4, **characterized in that** the mixture further contains salicylic acid.

6. Use according to claim 5 **characterized in that** the mixture contains 0.5 parts by weight of salicylic acid.

7. Use according to at least one of claims 1 to 6, **characterized in that** the mixture contains ethanol and/or isopropanol as a diluent.

## Revendications

1. Utilisation d'un mélange contenant de 0,1 à 1,0% en poids d'au moins une méthylxanthine et/ou au moins d'un de ses dérivés et de 0,1 à 0,25% en poids d'au moins un ester de l'acide nicotinique ainsi que des supports et/ou diluants habituels, ne contenant aucune substance active hormonale, pour préparer un médicament pour traiter l'alopécie androgénétique.

2. Utilisation selon la revendication 1 , caractérisée en ce que la méthylxanthine est la théophylline ou la caféine.

3. Utilisation selon la revendication 2, caractérisée en ce que l'ester de l'acide nicotinique est le nicotinate de benzyle.

4. Utilisation selon au moins une des revendications 1 à 3, caractérisée en ce que le mélange contient en outre des substances tampon, des stabilisateurs et des essences de parfums.

5. Utilisation selon au moins une des revendications 1 à 4, caractérisée en ce que le mélange contient en outre de l'acide salicylique.

6. Utilisation selon la revendication 2, caractérisée en ce que le mélange contient 0,5% en poids d'acide salicylique.

7. Utilisation selon au moins une des revendications 1 à 6, caractérisée en ce que le mélange contient comme diluant l'éthanol et/ou l'isopropanol.
